Europäisches Patentamt

European Patent Office

Office européen des brevets

(19)

(11) Publication number: **0 375 281**
**A2**

(12) **EUROPEAN PATENT APPLICATION**

(21) Application number: **89313082.3**

(22) Date of filing: **14.12.89**

(51) Int. Cl.5: **A61M 16/00**

(30) Priority: **23.12.88 GB 8830131**

(43) Date of publication of application:
**27.06.90 Bulletin 90/26**

(84) Designated Contracting States:
**DE FR GB SE**

(71) Applicant: **INSTRUMENTS AND MOVEMENTS LIMITED**
**18 St. George Street Hanover Square**
**London W1(GB)**

(72) Inventor: **Jones, Norman Stewart**
**46 Station Road Stanbridge**
**Leighton Buzzard Bedfordshire LU7 9JF(GB)**

(74) Representative: **Jack, Bruce James et al**
**FORRESTER & BOEHMERT**
**Widenmayerstrasse 4**
**D-8000 Munchen 22(DE)**

(54) **Demand valves.**

(57) A demand valve especially suitable for ventilator/resuscitator apparatus is characterised by a demand pressure sensor that is responsive both to demand pressure and to the rate of flow of the controlled fluid so that a valve opening product due to flow of the controlled fluid supplements the valve opening product due to demand pressure per se. In convenient embodiments the demand pressure sensor receives pressure signals from a pressure pickup in the controlled fluid flow path, this pickup having an orifice facing downstream or being in the throat of a choke of Venturi so as to generate a pressure signal having a static component due to demand pressure and a supplementary dynamic component due to flow of the controlled fluid.

EP 0 375 281 A2

## Demand Valves

Demand valves serve to control the flow of a fluid, usually a gas, from a source to a point of use and open to permit the flow in response to a sensed demand signalled by a pressure reduction on the downstream side of the valve. The most common utilisation of demand valves is in connection with the supply of breathing gas to divers, aircrew, users of breathing apparatus and persons undergoing anaesthesia, ventilation or resuscitation and while the invention is generally applicable to demand valves it is especially applicable to demand valves for ventilator/resuscitator apparatus.

An ideal demand valve, especially for this latter purpose, would satisfy all the demands of the user without any perceptible change in pressure at the point of use - for instance at a breathing mask. In practice, however, a certain pressure reduction ("demand pressure") at the point of use is necessary to achieve operation of the demand valve and for stability the demand pressure must increase with increasing demand flow. The usual design objective therefore is to provide a demand valve that opens at as low a demand pressure as is possible consistent with closing of the valve when the demand effort ceases, and in which the increase in demand pressure with flow is also minimal. The valve must be stable in operation - that is to say it must not "chatter" - and for many uses must be light, compact and unaffected by gravity and inertial effects.

It is usually impossible to reconcile all the requirements and although demand valves with very low opening demand pressures have been achieved, those in current use exhibit an undesirably high rate of demand pressure increase with increasing flow of the controlled fluid. One objective of the present invention is to overcome this disadvantage and to provide a demand valve that while meeting the discussed requirements has an especially low rate of demand pressure increase with controlled fluid flow rate.

Especially for ventilator/resuscitator apparatus, it is desirable to locate the demand valve remotely from the point of use, thereby to avoid encumbering the breathing mask or intratracheal tube with the demand valve mechanism. Typically the breathing mask or intratracheal tube is supplied with breathing gas from a source via a flexible pipe that may have a length of the order of one metre and that offers definite resistance to gas flow so that at high gas flow rates there is a significant pressure drop along its length. If the demand valve is directly connected to the upstream or source end of this pipe, the increasing pressure drop in the pipe as gas flow increases has to be matched by increasing demand pressure at the point of use, thereby exacerbating the above discussed problem. To circumvent this, it is known to control a remotely located demand valve by demand pressure ducted to it by a sensor pipe independent of the breathing gas flow pipe. However, the provision of a sensor pipe adds to the bulk and complexity of the system and provides another component that might fail (e.g. become disconnected) in use, with catastrophic results for the user. Such an arrangement also has the disadvantage of instability of operation, due to the extended feedback loop comprising the sensor pipe.

A further objective of the invention is therefore to provide a demand valve that may be located remotely from the point of use and sense demand pressure directly from the controlled fluid flow path without encountering performance degradation as a consequence pressure drop in the flow path between the point of use and the upstream sensing point.

In accordance with the invention, a demand valve has a demand pressure sensor adapted to respond both to demand pressure per se and to the controlled fluid flow rate in such manner that the valve-opening product of the sensor in response to demand pressure is supplemented by a valve-opening product due to flow of the controlled fluid.

The demand pressure sensor may be of any suitable construction. Its responsiveness to the controlled fluid flow may be directly attributable to its construction: for instance the demand pressure sensor may include sensing means directly influenced by controlled fluid flow through the demand valve. In preferred embodiments, however, the demand pressure sensor is indirectly responsive to controlled fluid flow through the demand valve, e.g. as a consequence of an arrangement whereby a control pressure sensed by the sensor comprises both a static component related to the demand pressure and a dynamic component related to the controlled fluid flow through the demand valve.

Thus in preferred embodiments of the invention, the demand pressure sensor has a pressure pickup arranged to be influenced by the controlled fluid flow through the valve. In one form, the demand pressure sensor pressure pickup comprises an orifice positioned in the controlled fluid flow path and facing downstream in the latter so that static pressure at the orifice is supplemented by a dynamic pressure related to the controlled fluid flow in said flow path.

In an alternative arrangement, the demand pressure sensor pressure pickup comprises an orifice in the throat of a choke or Venturi in the controlled fluid flow path, whereby the pressure at said orifice reduces with increasing controlled fluid flow velocity.

The invention is further explained with reference to the accompanying drawings in which

FIGURE 1 is a semi-diagrammatic illustration of the principal components of a two-stage demand valve to which the invention may be applied; and

FIGURES 2, 3 and 4 illustrate three possible ways in which the demand pressure sensor of a demand valve may be made responsive to mass flow of controlled fluid through the valve, in accordance with the invention.

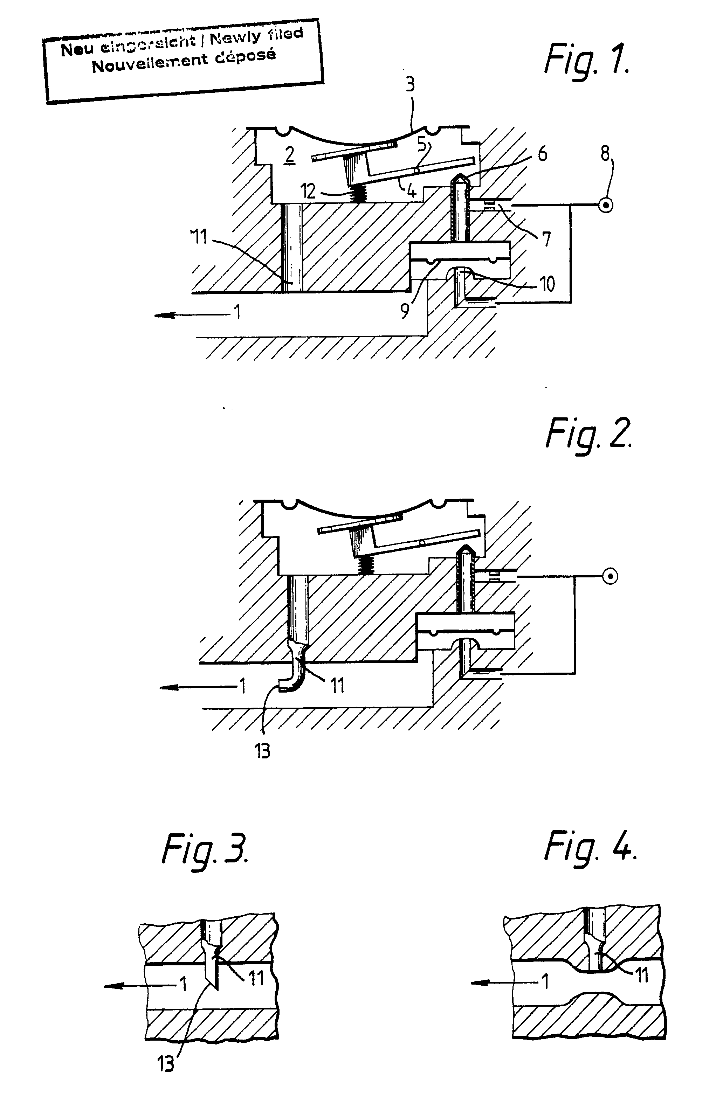

Figure 1 illustrates the principles of a typical two-stage demand valve such as used to control the flow of a breathing gas to a breathing mask. The demand valve has an outlet passage 1 and a demand pressure sensor comprising a sensing chamber 2 that is connected to the outlet passage 1 by a simple port 11 in the wall of the latter.

The demand pressure sensor further comprises a diaphragm 3 that moves in response to the pressure differential across it: typically the outer face of the diaphragm 3 is exposed to ambient pressure so that the diaphragm moves downwardly as seen in the drawing in response to a less-than-ambient pressure in the chamber 2. The diaphragm 3 is associated with a lever 4 pivoted at 5 and having a pad valve member that moves relatively to a nozzle 6 in response to rocking of the lever 4, the arrangement being such that the pad valve member moves away from the nozzle 6 when the lever 4 rocks in response to movement of the diaphragm 3 under the influence of decreasing pressure in the chamber 2. In Figure 1, the valve is shown with the diaphragm 3 deflected to move the pad valve away from the nozzle 6, as in response to less-than-ambient pressure in the sensing chamber 2.

The nozzle 6 is connected via a restrictor 7 to a breathing gas source shown symbolically at 8. The source 8 is further connected to an inlet passage extending to a control valve nozzle 10 that co-operates with a diaphragm 9 one side of which is exposed to the pressure in outlet passage 1 and the other side of which is exposed to the pressure at the nozzle 6.

A spring 12 biases the lever 4 to oppose opening movement of the pad valve member relative to the nozzle 6. Consequently in the absence of a pressure differential across the diaphragm 3 - that is to say, in the absence of a demand pressure - the nozzle 6 is obturated by the pad valve member and full source pressure builds up behind the nozzle 6 to deflect diaphragm 9 into valve-closing relationship to the valve nozzle 10, thereby to inhibit flow from the source 8 to the outlet passage 1.

A reduction in pressure at outlet passage 1, consequent upon an attempt at inhalation through a breathing mask connected thereto, appears as a corresponding reduction of pressure in the sensing chamber 2 and the diaphragm 3 is deflected downwardly as shown to rock the lever 4 so as to open the nozzle 6. Pressure behind the nozzle 6 accordingly reduces and the diaphragm 9 rises to open the valve nozzle 10 as shown and permit flow from the source 8 to the outlet passage 1. The size of the restrictor 7 is so chosen that when the nozzle 6 is fully open, the pressure drop across it due to the resultant flow is such that the diaphragm 9 can fully open the valve nozzle 10, to allow maximum breathing gas flow to the outlet passage 1. The proportionality of the system depends upon various factors including the diameters and stiffnesses of the diaphragms 3 and 9, the rate of spring 12 and the relative sizes of the nozzle 6 and restrictor 7.

In general it is relatively easy to design a demand valve of the configuration illustrated by Figure 1 and in which a low demand pressure in chamber 2 accomplishes initial opening of the valve. However if the size of the demand valve is to be minimised - often a design criterion - it is not possible to achieve also a large flow at a low demand pressure with reliable closing of the valve and stability of operation.

In accordance with the invention the demand pressure sensor of a demand valve is made responsive to the controlled fluid flow through the valve to supplement the response of the sensor to demand pressure per se. In a demand valve of the configuration illustrated by Figure 1 this may conveniently be accomplished by modifying the connection of the demand pressure sensing chamber 2 to the outlet passage 1 so that, in effect, the demand pressure pickup represented by the port 11 in Figure 1 is modified to produce in the sensing chamber 2 a control pressure that comprises a component due to the (static) demand pressure in the outlet passage 1 and a further (dynamic) component related to the gas flow in the outlet passage 1.

Figures 2, 3 and 4 illustrate different ways in which this objective can be achieved in practice. In the arrangement of Figure 2, the port 11 is substituted by a pickup pipe, that extends into the outlet passage 1 and is cranked so that its terminal portion extends axially of the latter to provide a downstream facing port 13 on the axis of passage 1. When gas flows in the passage 1, momentum exchange results in a reduced pressure appearing at the port 13 as compared with the static pressure in the passage 1.

In Figure 3 the pickup pipe is not cranked as in Figure 2 but has an obliquely cut end to provide a downstream facing port 13 to achieve a similar effect.

Figure 4 illustrates an application of Bernoulli's principle to achieve the desired effect. In this embodiment a choke or Venturi is formed in the outlet passage 1 and the port 11 is positioned at the throat of this. As flow velocity increases so the pressure at port 11 reduces as a consequence of the conservation of energy principle. Therefore the pressure at the port 11 is reduced in comparison with the static demand pressure downstream of the port.

With all these arrangements it follows that if a certain control pressure is required in the chamber 2 to achieve a certain demand flow in the outlet passage 1, other things being equal this control pressure can be achieved by means of the invention at a lower demand pressure than with conventional demand valve arrangements.

Moreover, it should be noted that if the demand valve is located remotely from the point of use so that pressure drop in the connection between the demand valve and the point of use occurs when gas flow commences, and increases with increasing gas flow, that pressure drop can be compensated by the arrangement of the invention, because increasing gas flow produces an increasing reduction of sensing chamber pressure relatively to the static pressure at the sensing point.

Accordingly, the demand valve of the invention is especially useful in ventilator/resuscitator apparatus in which location of the demand valve remote from the point of use (e.g. breathing mask) is desired, because it suffers none of the discussed problems of conventional demand valves when so used.

Although the invention has been illustrated in relation to its application to a two-stage demand valve it is of course equally applicable to all other forms of demand valve.

## Claims

1. A demand valve having a demand pressure sensor adapted to respond both to demand pressure and controlled fluid flow rate in such manner that the valve-opening product of the sensor in response to demand pressure is supplemented by a valve-opening product due to flow of the controlled fluid.

2. A demand valve according to claim 1, wherein the demand pressure sensor is indirectly responsive to controlled fluid flow.

3. A demand valve according to claim 2, having means to provide a control pressure to be sensed by the sensor and comprising a static component related to the demand pressure and a dynamic component related to the controlled fluid flow through the valve.

4. A demand valve according to claim 3, wherein the demand pressure sensor has a pressure pickup arranged to be influenced by the controlled fluid flow through the valve.

5. A demand valve according to claim 4, wherein said pressure pickup comprises an orifice positioned in the controlled fluid flow path and facing downstream in the latter.

6. A demand valve according to claim 4, wherein said pressure pickup comprises an orifice in the throat of a choke or Venturi in the controlled fluid flow path.

Fig. 1.

Fig. 2.

Fig. 3.

Fig. 4.